# EUROPÄISCHE PATENTSCHRIFT

(11) **EP 1 267 900 B1**
(45) Veröffentlichungstag und Bekanntmachung des Hinweises auf die Patenterteilung: **21.04.2004**
(21) Anmeldenummer: 01915417.8
(22) Anmeldetag: 26.03.2001
(51) Int. Cl.: A61K 35/78, A61K 7/48, A61P 17/00

(54) **EMULSION ENTHALTEND EINEN TRITERPEN-HALTIGEN PFLANZENEXTRAKT, VERFAHREN ZUR HERSTELLUNG DER EMULSION SOWIE ZUR GEWINNUNG DES PFLANZENEXTRAKTES**
EMULSION CONTAINING A TRITERPEN CONTAINING PLANT EXTRACT, METHOD FOR PRODUCING SAID EMULSION AND FOR OBTAINING A PLANT EXTRACT
EMULSION CONTENANT UN EXTRAIT DE PLANTE CONTENANT UN TRITERPENE, PROCEDE POUR PRODUIRE CETTE EMULSION ET POUR OBTENIR CET EXTRAIT DE PLANTE

(30) Priorität: 28.03.2000 DE 10015353; 16.11.2000 DE 10056902
(43) Veröffentlichungstag der Anmeldung: 02.01.2003
(73) Patentinhaber: BIRKEN GMBH, 75223 Niefern-Öschelbronn (DE)
(72) Erfinder: SCHEFFLER, Armin, 75223 Niefern-Öschelbronn (DE)
(74) Vertreter: Bickel, Michael
(86) Internationale Anmeldenummer: PCT/EP2001/003417
(87) Internationale Veröffentlichungsnummer: WO 2001/072315

(56) Entgegenhaltungen:
- WO-A-01/10885
- VILEGS JANETE H ET AL: "Extraction of low-polarity compounds (with emphasis on coumarin and kaurenoic acid) from Mikania glomerata ('guaco') leaves" BIOSIS, XP002163896
- ECKERMAN ET AL: "Comparison of Solvents for Extraction and Crystallization of Betulinol from Birch Bark Waste" PAPERCHEM2, XP002154153

## Beschreibung

Die vorliegende Erfindung betrifft ein Verfahren zur Gewinnung von Triterpenen aus Pflanzen und/oder deren Bestandteilen sowie eine Emulsion, deren wäßrige und fettige Phase durch den Pflanzenextrakt emulgiert wird, wobei der Pflanzenextrakt wenigstens ein Triterpen und/oder wenigstens ein Derivat eines Triterpens und die Emulsion weiterhin wenigstens ein Öl und/oder Fett und Wasser aufweist. Außerdem betrifft die vorliegende Erfindung ein Verfahren zur Herstellung der Emulsion sowie die Verwendung der Emulsionen zur Herstellung von Kosmetika und Arzneimitteln.

Natürliche Inhalts-, Aroma- und Wirkstoffe werden seit langer Zeit mittels verschiedener Extraktionstechniken aus vollständigen Pflanzen und deren Teilen, wie Blättern, Wurzeln, Früchten oder Rinde isoliert. Als einer der ersten Naturstoffe wurde Betulin, der Stoff, welcher der Birkenrinde ihre weiße Farbe verleiht, im Jahre 1788 aus Pflanzenmaterial gewonnen (Lowitz, M., Chemische Analysen, Hrsg. Crell, L., Vol. 2, S. 312). An diese erste Isolierung schlossen sich wissenschaftliche Studien einschließlich elementarer Analysen von Betulin an, die U. Hausmann 1876 publizierte (Hausmann, U., Annalen der Chemie, 182, S. 368).

Betulin-haltige Pflanzen sind im Pflanzenreich weit verbreitet. Sie gehören vorwiegend zu den Bedecktsamern (=Angiosperme Pflanzen), wobei in einigen Spezies der Ordnung Fagales (Buchenverwandte) und hier insbesondere in der Familie der Betulaceen (Birken) der Gehalt an Betulin in der äußeren Rinde kulminiert (Übersichtsartikel: Hayek, E. W. et al., (1989), A Bicentennial of Betulin, Phytochemistry, 28(9), S. 2229-2242). Der weiße Teil der Birkenrinde, insbesondere der Spezies Betula pendula, Betula verrucosa und Betula papyfera kann mehr als 30 % Betulin enthalten. Genauere Untersuchungen liegen von Rainer Ekman aus dem Jahre 1983 vor, wonach aus der trockenen äußeren Rinde von Betula verrucosa 21 - 40 % Triterpene bzw. 16,7 - 34 % Betulin extrahiert wurden (Ekman, R. (1983a), Holzforschung, 37, S. 205 - 211). Die innere Rinde enthält dagegen nur Spuren von Triterpenen im Bereich von ca. 0,37 - 0,43 % (Ekman, R. (1983b), Finn. Chem. Letters, S. 162).

Betulin ist ein pentazyklisches Triterpen mit einem Lupan-Gerüst, das auch als Betulinol, Trochoton, Birkenkampher und (Coryli-)resinol bezeichnet wird. Das charakteristische Merkmal der Lupan-Gruppe ist ein Ring mit fünf Kohlenstoffatomen innerhalb des pentazyklischen Systems, der eine α-Isopentenylgruppe an der Position C-19 besitzt. Betulin zeichnet sich weiterhin durch eine hohe thermische Stabilität aus, sein Schmelzpunkt liegt zwischen 250 und 261°C, wobei noch höhere Werte nach der Sublimation des rekristallisierten Produktes erhalten werden. Sein Molekulargewicht beträgt 442,7, es ist löslich in Pyridin und Tetrahydrofuran, aber nur gering löslich in Dichlormethan, Chloroform und kalten organischen Lösungsmitteln, wobei sich die Löslichkeit mit steigender Temperatur erheblich erhöht. In Wasser und kaltem Petrolether [Kohlenwasserstoffe mit 5 bis 8 C-Atomen (C₅-C₈ KW)] ist Betulin praktisch unlöslich. Kinetische Untersuchungen haben außerdem eine sehr geringe Reaktivität der Hydroxygruppen von Betulin gezeigt (Übersichtsartikel: Jääskeläinen, P. (1981), Paperi ja Puu - Papper och Trä 10, S. 599-603).

Bereits im Jahre 1899 hat J. Wheeler die antiseptischen Eigenschaften von Betulin nachgewiesen, es wurde daher für die Sterilisierung von Wundverbänden und Pflastern verwendet (Wheeler, J., (1899), Pharm. J., Die Darstellung des Betulin durch Sublimation, 494, Ref. Chem. Centr. 1900 I, S. 353). In der Naturmedizin und in der Volksheilkunde wurden und werden noch heute Abkochungen von Birkenrinde gewonnen, wobei meist jedoch nur der innere Teil verwendet wird, der nur Reste der weißen Birkenrinde und wegen mangelnder Löslichkeit wohl kein Betulin enthält, da der Gehalt an Betulin der inneren Rinde < 0,5 % beträgt und Betulin in Wasser oder Wasser-Alkohol Gemischen (bis 60 % Alkohol) unlöslich ist (Ekman, R. (1983b), supra).

Der so gewonnene Sud wird zur Behandlung von Wechselfieber, Wassersucht, Gicht und bei Hautkrankheiten sowie als Tinktur für Umschläge gegen Abszesse verwendet. Weiterhin wird Birkenrinde zur Herstellung des Birkenrindenöls, das auch zur Behandlung von Rheuma eingesetzt wurde und als Aromatikum verwendet (Hänsel, R. et al., (Hrsg.), (1994), Drogen A-D, Springer Verlag, Kapitel Betula, S. 502-511; Hayek, E. W. supra). Außerdem ist die Verwendung von Birkenextrakten als Badezusatz zur Behandlung speziell von Fußschweiß sowie als Zusatz zu Shampoos als Haarpflegemittel bekannt (Nowak, G. A., (1966), Cosmetic and Medicinal Properties of the Birch, Amer. Perfumer Cosmet., 81, S. 37). Jedoch sind auch hier nur wäßrige Auszüge aus Birkenblättern verwendet worden, die praktisch kein Betulin enthalten.

Neuere Untersuchungen lassen eine medizinische Wirkung von Betulin und Betulinderivaten vermuten. Im Tierversuch inhibierte Betulinsäure die Replikation von Retroviren, insbesondere des humanen Immundefizienz Virus (HIV1). Die beschriebenen bakteriostatischen und bakteriziden Wirkungen von Betulin gegen das Darmbakterium Escherichia coli, Salmonella typhi, Shigella flexneri und Staphylococcus aureus lassen auf eine breitere medizinische Anwendung schließen (Chen Sun, J. et. al., (1998), Anti-AIDS Agents, 32, Synthesis and anti-HIV activity of Betulin derivates, Bioorganic & Medical Chemistry Letters 8, S. 1267-1272; Evers, M. et. al., (1996), Betulinic acid derivates: a new class of human immunodeficiency virus type 1 specific inhibitors with a new mode of action, J. Med. Chem. 39, S. 1056-1068; Hayek, E. W. et. al., supra). Ferner konnte Betulin und Betulinderivaten eine entzündungshemmende, kortisonähnliche Wirkung ebenso wie eine zytostatische Wirkung bei Verwendung verschiedener Tumorzellinien in vitro zugewiesen werden (Carmen Recio, M., et. al. (1995), Investigations on the steroidal anti-inflammatory activity of triterpenoids from Diospyros leucomelas, Planta Med. 61, S. 9-12; Yasukawa, K., et. al., (1991), Sterol and triterpene derivates from plants (...), Oncogene 48, S. 72-76).

Für die Gewinnung von Betulin aus Birkenrinde, insbesondere aus dem Birkenkork kommt neben der Sublimation (Lowitz, M., supra) vor allem die Extraktion in siedenden Lösungsmitteln in Betracht. Als Lösungsmittel werden dabei hauptsächlich Alkohole und chlorierte Kohlenwasserstoffe verwendet (Ukkonen, K. und Erä, V., (1979), Kemia-Kemi 5, S. 217-220; Ekman, R. (1983a), supra; Eckermann, C. und Ekman, R. (1985), Paperi ja Puu - Papper och Trä 3, S. 100-106; O'Connell, M. M. et al., (1988), Phytochemistry 7, S. 2175-2176; Hua, Y. et al., (1991), Journal of Wood Chemistry and Technology 11(4), S. 503-516).

Nachteilig an der Sublimationstechnik ist die Tatsache, daß die Inhaltsstoffe nur mit sehr geringer Ausbeute erhalten werden, was den Einsatz von großen Ausgangsmengen erforderlich macht. Besonders nachteilig an der Sublimationstechnik ist außerdem das gleichzeitige Auftreten teerartiger Zersetzungsprodukte aus anderen Kork/Rindenbestandteilen, was ein mehrfaches Resublimieren oder Umkristallisieren erforderlich macht. Bei der Anwendung der Extraktionsmethode ist besonders nachteilig, daß Betulin in den genannten Lösungsmitteln relativ schlecht löslich ist und die Extraktion nur mit hohem Zeitaufwand durchgeführt werden kann. Auch hier ist mehrfaches Umkristallisieren erforderlich. Die besten Ergebnisse werden dabei mit höher siedenden Kohlenwasserstoffen erzielt (Eckermann, C. und Ekman, R. (1985), supra). Diese sind aber im Hinblick auf Kosmetika und Arzneimittel wegen der unvermeidbaren Lösungsmittelreste im Präparat besonders nachteilig.

Lösungsmittel, in denen Betulin gut löslich ist, wie beispielsweise Pyridin und Tetrahydrofuran, werden allgemein als giftig eingestuft. Sie scheiden aufgrund der gesundheitlichen Risiken und der nicht unerheblichen Handhabungsgefahren für die Extraktion in großem Maßstab aus. Ein weiterer Nachteil der genannten Lösungsmittel ist, daß erhebliche Mengen bräunlicher, unerwünschter Substanzen mit gelöst werden, deren spätere Abtrennung extrem aufwendig und unwirtschaftlich ist. Damit steht bis heute kein effizientes Verfahren zur Gewinnung von Betulin aus Birkenkork zur Verfügung, bei dem Betulin in großer Menge, mit einem hohen Reinheitsgrad und ohne Verwendung von stark gesundheitsgefährdenden Lösungsmitteln erhalten werden kann.

Im Stand der Technik sind weiterhin lediglich Verfahren zur Extraktion von lipophilen Naturstoffen unter hohem Druck und hoher Temperatur bekannt. Beispielsweise wird in der US 5,843,311 ein Verfahren zur Isolierung von organischen Stoffen mittels organischer Lösungsmittel beschrieben. Mit Hilfe dieses analytischen Verfahrens, das im kleinen Maßstab angewandt wird, können Proben auf Kontaminationen, Verunreinigungen oder Zusätze untersucht werden. Anwendung findet dieses Analyseverfahren vorwiegend zur Kontrolle in der Nahrungs- und Genußmittelindustrie, in der pharmazeutischen Industrie und bei der Analyse von Bodenproben. Nachteilig an diesem Verfahren ist seine auf den analytischen Maßstab beschränkte Anwendung.

In der US 5,647,976 ist ein Reaktionsgefäß beschrieben, das für die Extraktion von Inhaltsstoffen mittels Lösungsmittel unter erhöhtem Druck und erhöhter Temperatur verwendet werden kann. Dieses Reaktionsgefäß zeichnet sich durch einen Verschluß aus, der es erlaubt, Lösungsmittel in das Reaktionsgefäß einzuführen und aus ihm zu entfernen, ohne daß der Verschluß zerstört werden muß. Auf diese Weise werden Kontaminationen des Lösungsmittels wirkungsvoll verhindert. Ferner kann das Reaktionsgefäß einfach gehandhabt werden, so daß die Verwendung auch ungeschultem Personal offensteht. Die US 5,647,976 beschreibt lediglich das genannte Reaktionsgefäß, jedoch ergeben sich aus ihr keine Hinweise für die Durchführung eines Extraktionsverfahrens. Sie enthält außerdem keine Angaben über die Qualität und Reinheit der in dem Reaktionsgefäß isolierten Inhaltsstoffe. Die US 5,660,727 beschreibt die Verwendung des Reaktionsgefäßes aus der US 5,647,976 in einer automatischen Rotationseinheit, welche die gleichzeitige Analyse von mehreren Proben erlaubt. Das Volumen der Reaktionsgefäße, die in die Rotationseinheit eingesetzt werden können, liegt zwischen 10 und 30 ml. Daher ist die Anwendung dieser Gegenstände auf den Analysemaßstab beschränkt und nicht für den Einsatz im großtechnischen Rahmen geeignet.

In der US 5,785,856, die eine Teilanmeldung der US 5,660,727 darstellt, ist neben der Rotationseinheit auch ein Verfahren. zur Extraktion von Inhaltsstoffen unter Verwendung von Lösungsmitteln beschrieben. Das Verfahren wird unter erhöhtem Druck und erhöhter Temperatur durchgeführt. Hierfür wird das Reaktionsgefäß mit zu analysierendem Probenmaterial befüllt, in die Rotationseinheit eingesetzt und automatisch mit Lösungsmittel versetzt. Anschließend wird der Druck und die Temperatur auf vorgegebene Werte erhöht. Nach erfolgter Extraktion wird das Lösungsmittel in einen Aufnahmebehälter abgeführt. Das Reaktionsgefäß einschließlich der Zuführ- und Abführleitungen kann mit einem inerten Gas wie beispielsweise Stickstoff zur Reinigung gespült werden. Nachteilig an diesem Verfahren ist insbesondere, daß es sich zwar zum Nachweis von lösbaren Stoffen in einer Analyseprobe, aber nicht oder nur schlecht für die präparative Darstellung und Gewinnung von Inhaltsstoffen in großer Menge und Reinheit eignet, weil sich gerade hierbei Kontaminationen der Probe sehr störend auf den Reinheitsgrad des extrahierten Inhaltsstoffs auswirken. Ferner ist das in der US 5,785,856 beschriebene Verfahren aufgrund seiner Konzeption auf den analytischen Maßstab beschränkt. Das Dokument enthält keine Angaben über erzielbare Reinheitsgrade und die Möglichkeit zum großtechnischen Einsatz.

Alle im Stand der Technik beschriebenen Verfahren arbeiten diskontinuierlich, d.h. eine bestimmte Menge wird jeweils in einem zu befüllenden und anschließend zu entleerenden Gefäß extrahiert. Bisher ist kein kontinuierliches Verfahren verwirklicht worden, bei dem das Pflanzenmaterial und das frische Lösungsmittel kontinuierlich zugeführt, die Extraktion im Gegenstromverfahren durchgeführt und das extraktgesättigte Lösungsmittel sowie das vollständig extrahierte Pflanzenmaterial gleichmäßig abgeführt werden.

Im Stand der Technik wurden außerdem bereits einige Versuche unternommen, die oberflächenaktiven Eigenschaften von Betulin zu modifizieren, um ein technisch anwendbares Produkt herzustellen. So hatte beispielsweise Pasich bereits in den 60er Jahren Betulin und seine Ester, wie Succinate, Phtalate und Tetrachlorphtalate als Emulgatoren für weiße Vaseline, Waltran und Erdnußöl untersucht und war zu dem Schluß gekommen, daß Betulin hinsichtlich seiner emulgierenden Eigenschaften, d.h. seiner Fähigkeit wäßrige und ölige Komponenten miteinander zu verbinden, mit bekannten technischen Emulgatoren vergleichbar war. Seine Betulin-haltigen Emulgatoren waren jedoch wenig stabil. Er verwendete umkristallisiertes Betulin und erreichte keine ausreichende Stabilität der Emulsionen. Am günstigsten waren noch die Phtalate und Tetrachlorphtalate, die unter dem Aspekt besserer Wasserlöslichkeit hergestellt wurden. Die Emulsionen schieden nach kurzer Zeit ölige Schichten ab, was auf eine ungenügende Stabilität hinweist. Außerdem wurden die beschriebenen Emulsionen mit antimikrobiell wirksamen Konservierungsstoffen versetzt, wobei vorwiegend Benzoesäurederivate verwendet wurde (Pasich, J., (1965), Triterpenoid emulsifiers of plant origin V. Emulsifying properties of Betulin and certain of its esters. Farm. Polska 21, Nr. 17-18, S. 661-666).

Doch gerade der Zusatz von Konservierungsstoffen, die ihrerseits allergene und toxische Wirkungen hervorrufen können, macht den positiven, therapeutischen Effekt des Betulins besonders auf geschädigter Haut vielfach zunichte, so daß die in der Literatur bekannten Präparationen von Betulin-haltigen Emulsionen nicht oder nur begrenzt in der modernen Hautpflege angewendet werden können.

Diese Nachteile im Stand der Technik haben trotz der in großen Mengen bei der Holzgewinnung als Abfallprodukt anfallen-' den Birkenrinde bis heute zu keiner nennenswerten technischen Aufarbeitung, Nutzung oder Verwertung der im Birkenkork enthaltenen Triterpene, insbesondere des in großen Mengen enthaltenen Betulins geführt.

Die Mengen derzeit ungenutzten Betulins sind gewaltig. Allein in einer einzigen Zellstoffabrik in Finnland (UPM Kymmene, Lappeenranta, Finnland) wird eine extrahierbare Menge von ca. 4.000 bis 5.000 t Betulin pro Jahr verbrannt. In Schweden, Finnland, Rußland und Kanada sind zahlreiche Zellstoffabriken dieser Größenordnung tätig. Eine wirtschaftliche Nutzung dieses nachwachsenden Rohstoffes steht und fällt mit einem wirtschaftlichen Gewinnungsverfahren, das bisher nicht beschrieben ist und das durch die vorliegende Erfindung zur Verfügung gestellt wird.

Der vorliegenden Erfindung liegt daher zunächst die Aufgabe zugrunde, ein Verfahren vorzuschlagen, das unter vertretbarem Lösungsmittelverbrauch die Gewinnung von großen Mengen an Triterpenen mit hohem Reinheitsgrad sowohl im Chargenbetrieb als auch kontinuierlich erlaubt. Mit Hilfe des Verfahrens soll außerdem eine Möglichkeit eröffnet werden, die in der Zellstoffindustrie als Abfallprodukt anfallende Birkenrinde zu verwerten. Zudem soll das Verfahren einfach, kostengünstig und schnell durchführbar sein. Außerdem soll gewährleistet sein, daß die Restlösungsmittelmenge im Extrakt den Bestimmungen des Europäischen Arzneibuches entspricht, hier insbesondere der "Guideline for Residual Solvents": ICH Q3C-Impurities (ICH = International Conference on Harmonization of technical requirements for registration of pharmaceuticals for human use). Der vorliegenden Erfindung liegt außerdem die Aufgabe zugrunde, eine Triterpen-haltige Emulsion zur Verfügung zu stellen, die pharmazeutisch aktive Komponenten aufweist und gleichzeitig ohne den Zusatz von Konservierungsstoffen über einen längeren Zeitraum ausreichend stabil ist.

Zudem soll die Emulsion auf einfache Weise hergestellt werden können.

Die erste Aufgabe wird dadurch gelöst, daß bei dem erfindungsgemäßen Verfahren zur Gewinnung von Triterpenen aus Pflanzen und/oder deren Bestandteilen die Pflanzenteile zunächst bei 20°C bis 70°C mit einem Lösungsmittel gewaschen werden, in dem die Triterpene nicht oder nur gering löslich sind.

Anschließend werden die Triterpene mit einem Lösungsmittel unter erhöhtem Druck und erhöhter Temperatur extrahiert. Daran schließt sich die Abkühlung und gleichzeitige Entspannung der die Triterpene enthaltenden Lösung an, wobei die Triterpene in dem Lösungsmittel in extrem kleinen Partikeln ausfallen und weitere Triterpene bei fortschreitender Abkühlung an diesen Partikeln auskristallisieren. Um die Reinheit der Triterpene zusätzlich zu erhöhen, werden sie nach der Filtration bei Raumtemperatur mit einem frischen Lösungsmittel gewaschen. Durch dieses Verfahren wird insbesondere erreicht, daß Triterpene auf einfache Weise im großtechnischen Maßstab gewonnen werden können.

Im Rahmen der vorliegenden Erfindung bedeutet der Ausdruck "Waschen (...) in einem Lösungsmittel, in dem die Triterpene (...) nicht oder nur gering löslich sind", daß die Löslichkeit der Triterpene nicht mehr als 1 g/Liter beträgt. Durch diesen ersten Waschschritt werden vorteilhafterweise leichter lösliche Verunreinigungen entfernt.

Die Extraktion der Triterpene erfolgt mit einem Lösungsmittel unter erhöhtem Druck und erhöhter Temperatur, gegebenenfalls im überkritischen Bereich. Hierfür können als Lösungsmittel beispielsweise überkritisches Kohlendioxid sowie Kohlenwasserstoffe im überkritischen Bereich als auch flüssige Kohlenwasserstoffe unter erhöhtem Druck oder Gemische verschiedener Kohlenwasserstoffe verwendet werden.

Vor dem Waschen [Schritt (e)] muß der Filterkuchen vorteilhafterweise nicht in einem weiteren Verfahrensschritt getrocknet werden. Dadurch ist das Verfahren besonders einfach durchzuführen.

Das Waschen der Triterpene [Schritt (e)] selbst kann außerdem, um das erfindungsgemäße Verfahren besonders wenig aufwendig und kostengünstig zu gestalten, unter Normalbedingungen durchgeführt werden.

In einer besonders bevorzugten Ausführungsform wird das erfindungsgemäße Verfahren kontinuierlich durchgeführt. Dazu wird das Pflanzenmaterial und kaltes Lösungsmittel durch Pumpen in ein druckfestes Rohr gefördert und im Gegenstrom bei 20°C bis 70°C gewaschen. Durch ein zweites Pumpsystem wird das gewaschene Pflanzenmaterial in ein zweites, ebenfalls druckfestes und heizbares Rohr gefördert und im Gegenstrom mit frischem, überkritisch erhitztem oder flüssigem Lösungsmittel extrahiert. Die Ausleitung der Wasch- und der Extraktlösung erfolgt jeweils über ein Filtersystem. Die Pflanzenteile werden aufgrund ihres Dichteunterschiedes gegen den Lösungsmittelstrom geführt und am entgegengesetzten Ende vom Lösungsmittelauslaß ausgeschleust. Die Extraktlösung wird - unter Aufrechterhaltung des Druckes im Extraktionssystem - über ein Ventilsystem Sprühdüsen zugeführt und erst hier entspannt.

Birkenrinde steht als kostengünstiger Rohstoff insbesondere als Abfallprodukt der Zellstoffindustrie in großen Mengen zur Verfügung. In einer bevorzugten Ausführungsform der vorliegenden Erfindung werden die Triterpene aus Birkenrinde, vorzugsweise aus dem weißen Teil der Birkenrinde, der auch als Birkenkork bezeichnet wird, gewonnen. "Separierter Birkenkork" ist sowohl die von Hand abgezogene weiße äußere Schicht, die als Korkhaut die Stämme von Birken nach außen abschließt und daher außerhalb der eigentlichen Rinde liegt, als auch der nachträglich beispielsweise mit Hilfe einer Hammermühle und eines Flotationsverfahrens von einer Gesamtschälung abgetrennte (= separierte) Birkenkork.

In einer besonders bevorzugten Ausführungsform werden die Triterpene in einem Reinheitsgrad von mindestens 80 %, vorzugsweise 85 %, insbesondere 90 %, besonders bevorzugt von über 90 % erhalten.

Der Hauptbestandteil der extrahierten Triterpene ist in einer weiteren Ausführungsform Betulin, wobei sein Gehalt mindestens 80 %, vorzugsweise 85 %, insbesondere 90 %, besonders bevorzugt über 90 % beträgt.

Es ist außerdem vorteilhaft, für den ersten Waschschritt, für die eigentliche Extraktion und für den abschließenden Waschschritt das gleiche Lösungsmittel zu verwenden. Auf diese Weise können die besten Reinheitswerte erzielt werden. Zudem ist das Verfahren bei Verwendung nur eines Lösungsmittels besonders einfach durchzuführen.

Als Lösungsmittel eignet sich besonders ein niedrig siedender Kohlenwasserstoff oder ein Gemisch, das einen niedrig siedenden Kohlenwasserstoff aufweist, weil ein niedrig siedender Kohlenwasserstoff anschließend sehr einfach entfernt werden kann. Der Siedepunkt des verwendeten Kohlenwasserstoffs, bzw. des Gemisches liegt dabei vorzugsweise unter 100°C. Besonders bevorzugt ist die Verwendung von n-Pentan, n-Hexan oder n-Heptan, die alle kostengünstig sind, keine speziellen gesundheitlichen Risiken bergen und in ausreichenden Mengen in guter Qualität zur Verfügung stehen.

Es hat sich als besonders vorteilhaft herausgestellt, für den ersten Waschschritt das Lösungsmittel zu verwenden, das als Filtrat nach der Hauptextraktion anfällt. Auf diese Weise werden grobe Verunreinigungen des Ausgangsmaterials vorab wirksam entfernt, ohne zusätzlich frisches Lösungsmittel einsetzen zu müssen. Diese Kontaminationen oder Nebenbestandteile würden bei der Extraktion den Reinheitsgrad negativ beeinflussen. Um den Reinheitsgrad weiter zu steigern, können die extrahierten Inhaltsstoffe ferner durch frisches Lösungsmittel abschließend nachgewaschen werden.

In einer bevorzugten Ausführungsform wird der erste Waschschritt unter einem Druck von 1 bis 300 bar, vorzugsweise 10 bis 35 bar, besonders bevorzugt 25 bar durchgeführt. Die eigentliche Extraktion erfolgt vorteilhafterweise bei einer Temperatur von 50 bis 200°C, vorzugsweise 140 bis 160 °C, besonders bevorzugt 150°C und unter einem Druck von 1 bis 300 bar, vorzugsweise 10 bis 35 bar, besonders bevorzugt 25 bar. Bei diesen Extraktionsbedingungen werden große Mengen an reinen Triterpenen, insbesondere reines Betulin erhalten.

Das erfindungsgemäße Verfahren hat sich als besonders vorteilhaft erwiesen, wenn die Auskristallisierung der Triterpene als Mikrokristallisation mit einer durchschnittlichen Korngröße von < 40 µm, insbesondere von 2 bis 32 µm, erfolgt.

Ein weiterer Gegenstand der vorliegenden Erfindung ist eine Emulsion, deren wäßrige und fettige Phase durch einen Pflanzenextrakt emulgiert wird, wobei der Pflanzenextrakt wenigstens ein Triterpen und/oder wenigstens ein Derivat eines Triterpens und die Emulsion weiterhin wenigstens ein Öl und/oder Fett und Wasser aufweist.

Das Triterpen und/oder sein Derivat konserviert und emulgiert die Emulsion. Es ist zudem pharmazeutisch aktiv. Dadurch wird insbesondere erreicht, daß die Emulsion keine zusätzlichen Konservierungsstoffe aufweist, wodurch sie einen besonders hohen Reinheitsgrad und eine gute Verträglichkeit vor allem bei problematischen Applikationen auf geschädigter Haut besitzt.

Der Pflanzenextrakt ist dabei ein Extrakt aus Birkenrinde, vorzugsweise aus dem weißen Teil der Birkenrinde, die auch als Birkenkork bezeichnet wird.

In einer weiteren Ausführungsform enthält der Pflanzenextrakt wenigstens eine der Substanzen Betulin, Betulinsäure, Lupeol, Erythrodiol, Allobetulin, Phellonsäure, Hydroxylakton, Betulinaldehyd, β-Amyrin, Oleanolsäure, Ursolsäure, verestertes Betulin und/oder β-Sitosterol. Diese Pflanzeninhaltsstoffe können sowohl einzeln als auch miteinander in verschiedenen Kombinationen in der erfindungsgemäßen Emulsion verwendet werden. Eine besonders geeignete Kombination besteht aus Betulin > 80 %, Betulinsäure < 10 %, Lupeol < 3 %, Oleanolsäure < 4 %, Erythrodiol < 4 % und Wasser < 1 %.

Die Emulsion enthält das Triterpen und/oder sein Derivat vorzugsweise in einer Konzentration von 2 bis 10 %. In Abhängigkeit von der gewünschten Konsistenz kann der Anteil des Triterpens und/oder seines Derivates im genannten Konzentrationsbereich variiert werden. Für die Verwendung in einer flüssigeren Lotion weist die Emulsion beispielsweise eine Konzentration des Triterpens und/oder seines Derivates von 2 bis 3,5 % auf. Wird die Emulsion in einer Creme verwendet, beträgt die Konzentration des Triterpens und/oder seines Derivates vorzugsweise zwischen 3,5 und 10 %.

Der ölige und/oder fettige Bestandteil der Emulsion kann aus allen in der Dermatologie und Kosmetik bekannten Ölen und Fetten ausgewählt werden. Es eignen sich besonders tierische und pflanzliche Fette wie Jojobaöl, Erdnußöl und Olivenöl. Besonders bevorzugt ist die Verwendung von Avocado und/oder Mandelöl.

Um die erfindungsgemäße Emulsion in ihrer Konsistenz zu regulieren, können herkömmliche Feuchthalte- und/oder Verdickungs- und Festigungsmittel zugesetzt werden. Als Feuchthaltemittel können insbesondere Glycerin und Harnstoff verwendet werden. Dabei eignen sich vor allem Konzentrationen von jeweils 3 bis 10 % Glycerin und/oder Harnstoff bezogen auf die Menge der Emulsion. Als Verdickungsmittel kann beispielsweise ein Polysaccharid verwendet werden, das in einer Konzentration von 0,2 bis 2 %, vorzugsweise 0,5 %, bezogen auf die Menge des eingesetzten Wassers, hinzugefügt wird. Natürliche Polysaccharide sowie weitere Zuckerverbindungen und ihre Derivate eignen sich besonders gut, weil sie nicht mit den weiteren Komponenten der Emulsion reagieren, sie insbesondere nicht verändern und damit chemisch inert sind.

Daher kann das Polysaccharid Agar Agar sehr gut verwendet werden. Agar Agar ist ein Gemisch aus Agarose und Agaropectin, das aus Rotalgen gewonnen wird und den Vorteil besitzt, daß es von den meisten Mikroorganismen nicht abgebaut werden kann. Alternativ dazu kann das Polysaccharid Carrageenan verwendet werden, das natürlicherweise in der Zellwand von Rotund einigen Braunalgen vorkommt und die gleichen Vorteile wie Agar Agar besitzt.

In einer weiteren Ausführungsform weist die Emulsion 2 bis 10 % Extrakt aus dem weißen Teil der Birkenrinde, 20 bis 30 % Avocadoöl, 10 bis 20 % Mandelöl und 40 bis 68 % Wasser auf. Die am meisten bevorzugten Konzentrationen betragen 4 % Extrakt aus dem weißen Teil der Birkenrinde, 29,3 % Avocadoöl, 14,7 % Mandelöl und 52 % Wasser.

Eine andere erfindungsgemäße Emulsion enthält 2 bis 10 % Extrakt aus dem weißen Teil der Birkenrinde, 20 bis 30 % Avocadoöl, 10 bis 20 % Mandelöl, 5 bis 10 % Feuchthaltemittel und 30 bis 63 % Wasser. Hierbei werden die Konzentrationen 4 % Extrakt aus dem weißen Teil der Birkenrinde, 29,3 % Avocadoöl, 14,7 % Mandelöl, 5 % Glycerin und/oder 5 % Harnstoff und 42 bis 47 % Wasser am meisten bevorzugt.

Der Extrakt aus dem weißen Teil der Birkenrinde, der in allen Emulsionen enthalten ist, weist mindestens 80 % Betulin, maximal 10 % Betulinsäure, maximal 3 % Lupeol und maximal 4 % Oleanolsäure auf.

Die Emulsion eignet sich aufgrund ihrer Zusammensetzung und der nachgewiesenen Ungiftigkeit hervorragend als Salbengrundlage zum Einrühren aller dem Fachmann bekannten Duft-, Kosmetik- und Arzneistoffe.

Ein weiterer Gegenstand der vorliegenden Erfindung ist ein Verfahren zur Herstellung der erfindungsgemäßen Emulsion, wobei das Verfahren die Schritte umfaßt:
(a) Dispergieren des Triterpens in einem Öl und/oder Fett;
(b) Emulgieren von Wasser;
(c) Homogenisieren der Emulsion bis zur erforderlichen Konsistenz.

Dabei erfolgt das Dispergieren und Homogenisieren vorteilhafterweise in einem Homogenisiermischer, weil das Verfahren auf diese Weise besonders einfach durchgeführt werden kann. Ferner können auch kleine Mengen an Emulsion für die individuelle Verwendung zubereitet werden.

In einer besonderen Ausführungsform wird dem Wasser, das bei dem Emulgieren verwendet wird, ein Feuchthaltemittel, insbesondere Glycerin und/oder Harnstoff, oder ein Verdickungsmittel, insbesondere ein Polysaccharid zugesetzt. Dadurch kann eine besonders cremige Konsistenz erreicht werden.

Ein weiterer Gegenstand der vorliegenden Erfindung ist die Verwendung der erfindungsgemäßen Emulsionen zur Herstellung eines Kosmetikums, vorzugsweise in Form einer Salbe, einer Lotion, einer Creme, eines Gels, eines Gelees oder eines Shampoos, insbesondere zum Auftragen auf die Haut, die Kopfhaut und/oder zum Inhalieren. Aufgrund ihrer chemischen Verwandtschaft mit Sterolen eignen sich die Triterpene, insbesondere das gewonnene Betulin zur Haut- und Kopfhautpflege. Es beruhigt und glättet die Haut, mindert den Wasserverlust und beugt Reizungen und Rötungen vor. So kann das Kosmetikum gut zur Feuchthaltung und Glättung der Haut sowie zur Verminderung von Altersflecken und bei schuppender Haut eingesetzt werden. Ein weiterer Vorteil ist die Wasserunlöslichkeit der Triterpene, so daß Haut, die stark und lange dem Wasser ausgesetzt ist, besonders gut geschützt wird. Aufgrund der antibakteriellen Wirkung ist die Verwendung als Desodorant besonders vorteilhaft. Hinzu kommt eine Verminderung der Transpiration aufgrund eines wirksamen Verschließens der Schweißdrüsen.

Ein weiterer Gegenstand der vorliegenden Erfindung ist die Verwendung der erfindungsgemäßen Emulsionen zur Herstellung eines Arzneimittels, vorzugsweise in Form einer Salbe, einer Lotion, einer Creme, eines Gels, eines Gelees oder eines Shampoos, insbesondere zum Auftragen auf die Haut, die Kopfhaut und/oder zum Inhalieren, besonders bevorzugt bei dermatologischen Veränderungen der Haut und/oder der Kopfhaut, vor allem bei Neurodermitis, Psoriasis, seborrhoischem Ekzem, Melanodermie, Präcancerosen der Haut sowie zum Inhalieren bei asthmatischen Anfällen und/oder zur Substitution von Glucocorticoiden. Ebenfalls aufgrund der großen Reinheit sind die gewonnenen Triterpene, insbesondere das gewonnene Betulin für den therapeutischen Einsatz bei vorgeschädigter Haut geeignet.

Die erfindungsgemäßen Emulsionen sind für den oben genannten Einsatz so gut geeignet, weil der Extrakt aus dem weißen Teil der Birkenrinde keine mutagenen Eigenschaften aufweist. Topische Toxizitätstests wurden ebenfalls durchgeführt, wobei sich keinerlei sensibilisierende Eigenschaften zeigten.

Die nachfolgend beschriebenen Beispiele dienen der Erläuterung und dem besseren Verständnis der vorliegenden Erfindung, sie sollen diese aber nicht einschränken.

### BEISPIELE

### I. Anwendungsbeobachtungen

Es wurden verschiedene Anwendungsbeobachtungen dermatologischer Hautveränderungen von niedergelassenen Ärzten sowie Klinikärzten durchgeführt. Nach der Diagnose durch die Ärzte erfolgte eine Behandlung der Patienten mit der erfindungsgemäßen Emulsion, die nach dem beschriebenen Verfahren hergestellt worden war.

### Beispiel 1:

- Patient:: männlich, 68 Jahre
- Diagnose:: schwere, generalisierte Psoriasis vulgaris
- Vorbehandlung:: mittelstarke Kortisonsalbe
- Anwendung:: Gesicht
- Behandlung:: Mehrmaliges tägliches Auftragen der erfindungsgemäßen Emulsion als Salbenpräparat auf die betroffenen Hautstellen.
- Resultat:: Auftreten von Psoriasiseffloreszenzen nach Absetzen der Kortisonsalbe wurden durch die Anwendung der erfindungsgemäßen Emulsion verhindert.

### Beispiel 2:

- Patient:: männlich, 48 Jahre
- Diagnose:: leichte Psoriasis vulgaris
- Vorbehandlung:: mittelstarke Kortisonsalbe
- Anwendung:: Kopf
- Behandlung:: Mehrmaliges tägliches Auftragen der erfindungsgemäßen Emulsion als Salbenpräparat auf die betroffenen Hautstellen.
- Resultat:: Abheilung der ca. 5 DM-Stück großen prästernalen entzündlichen Herde am Kopf bei Anwendung der erfindungsgemäßen Emulsion innerhalb von 14 Tagen; Wiederaufflammen der Entzündung (=Rezidiv) nach Absetzen der Emulsion; erneute deutliche Besserung bei Wiederaufnahme der Behandlung.

### Beispiel 3:

- Patient:: weiblich, 5 Monate
- Diagnose:: Neurodermitis, hyperkerakotische, vorgealterte, rissig-schrundige Haut
- Anwendung:: Körper
- Behandlung:: Mehrmaliges tägliches Auftragen der erfindungsgemäßen Emulsion als Salbenpräparat auf die betroffenen Hautstellen.
- Resultat:: Sehr deutliche Besserung, hervorragende Wirksamkeit bei gleichzeitiger guter Verträglichkeit.

### Beispiel 4:

- Patient:: weiblich, 10 Jahre
- Diagnose:: Neurodermitis, hyperkeratotische, vorgealterte, rissig-schrundige Haut
- Anwendung:: Körper
- Behandlung:: Mehrmaliges tägliches Auftragen der erfindungsgemäßen Emulsion als Salbenpräparat auf die betroffenen Hautstellen.
- Resultat:: Sehr deutliche Besserung, hervorragende Wirksamkeit bei gleichzeitiger guter Verträglichkeit.

### Beispiel 5:

- Patient:: männlich, 5 Monate
- Diagnose:: Neurodermitis, hyperkeratotische, vorgealterte, rissig-schrundige Haut
- Anwendung:: Körper
- Behandlung:: Mehrmaliges tägliches Auftragen der erfindungsgemäßen Emulsion als Salbenpräparat auf die betroffenen Hautstellen.
- Resultat:: Sehr deutliche Besserung, hervorragende Wirksamkeit bei gleichzeitiger guter Verträglichkeit.

### Beispiel 6:

- Patient:: weiblich, 28 Jahre
- Diagnose:: Neurodermitis, neurodermitische Effloreszenzen
- Anwendung:: Ellenbeugen, Gesicht, Dekolleté
- Vorbehandlung:: Dermatodoron, Stibium metallicum-Salbe, Top-Isolon-Bartel-Salbe, Mesembryanthemum-Salbe; unter Anwendung dieser Salben kam es zu keiner durchgreifenden Besserung der neurodermitischen Stellen.
- Behandlung:: Mehrmaliges tägliches Auftragen der erfindungsgemäßen Emulsion als Salbenpräparat auf die betroffenen Hautstellen.
- Resultat:: Deutliche Reduktion der Neurodermitis

### Beispiel 7:

- Patient:: weiblich, 79 Jahre
- Diagnose:: Seborrhoisches Ekzem
- Anwendung:: Kopf
- Behandlung:: Mehrmaliges tägliches Auftragen der erfindungsgemäßen Emulsion als Salbenpräparat auf die betroffenen Hautstellen.
- Resultat:: Reduktion der Schorfbildung im Schädelbereich, rötliche Areale verblassten etwas langsamer.

### Beispiel 8:

- Patient:: weiblich, 55 Jahre
- Diagnose:: stark juckendes, schuppendes, nässendes Ekzem
- Anwendung:: Gesicht, Kopf
- Behandlung:: Mehrmaliges tägliches Auftragen der erfindungsgemäßen Emulsion als Salbenpräparat auf die betroffenen Hautstellen.
- Resultat:: Sichtbare Besserung der behandelten Stellen

### Beispiel 9:

- Patient:: weiblich, 33 Jahre
- Diagnose:: trockenes, juckendes, lichenifiziertes, schorfiges Ekzem
- Anwendung:: Unterschenkel
- Behandlung:: Mehrmaliges tägliches Auftragen der erfindungsgemäßen Emulsion als Salbenpräparat auf die betroffenen Hautstellen.
- Resultat:: noch nicht bewertet

### II. Herstellung des Pflanzenextraktes

### Ausgangsmaterial: Birkenkork

### A) Beschreibung und Qualitätssicherung

Der Birkenkork wird geliefert von:
1. der "wood pulp company Kaukas" (UPM-Kymmene Lappeenranta, Finnland).
   Kaukas trennt die Rinde mechanisch von dem Birkenholz und auch der Kork wird mechanisch von der inneren Rinde gelöst.
2. durch eigene Mitarbeiter, die den Kork von Hand schneiden.
   In einem ersten Schritt werden der mechanisch getrennte und der handgeschnittene Kork bezüglich seiner Identität analysiert. Zu diesem Zweck wird der Kork mikroskopisch untersucht, wobei sich mehrere individuelle Korkschichten und Lentizellen, die für Birke typisch sind, zeigen. Zusätzlich wird die extrahierbare Menge an Triterpenen mit n-Hexan bei 140 bis 160°C unter steigendendem Druck untersucht. Die extrahierbare Menge an Triterpenen mit dem Hauptbestandteil Betulin übersteigt 10 %, weil nur im Kork von Birken mit weißen Rinden solche Mengen vorkommen. Die Reinheitskontrollen des Materials schließen eine Analyse hinsichtlich Aflatoxinen, Schwermetallen, Herbiziden und Pestiziden gemäß europäischen Standards ein.

### B) Zerkleinern des Korks

Der Kork wird in einer Schneidmühle gemahlen (Firma Retsch) und durch ein Sieb mit einer Porengröße von 1 mm Durchmesser aus der Maschine abgesaugt. Für die weitere Extraktion werden nur Partikel mit einer Korngröße ≤ 1 mm verwendet.

### Extraktion der Triterpene:

Für die Extraktion wird das Phänomen ausgenutzt, daß Triterpene aus Birkenkork in kaltem n-Hexan praktisch unlöslich sind, sich aber in heißem n-Hexan (140 bis 160°C) unter Druck gut lösen. Dieses Verfahren wird in 2 Schritten durchgeführt:
1. Der Kork wird in einem Soxhlett Apparat nach dem Soxhlett Verfahren mit n-Hexan gereinigt (heiß, < Siedepunkt 69°C) bis die n-Hexan Lösung nicht mehr gelblich ist.
2. Der aktive Bestandteil wird aus den vorgewaschenen Korkgranulaten mit n-Hexan bei 140 bis 160°C unter erhöhtem Druck extrahiert. (Extraktion mittels Dionex ASE 300 mit 100 ml Gefäßen). Durch die Freisetzung der überhitzten Lösung enthaltend n-Hexan und Triterpene, erfolgt eine unmittelbare Kristallisation, so daß ein mikrokristallines Pulver aus Triterpenen direkt in kaltem n-Hexan erhalten wird. Dieses Pulver wird gefiltert und zweimal in kaltem n-Hexan gewaschen. Die Triterpene werden bei 80 bis 100°C getrocknet.

### Bestimmung der Reinheit des Extraktes:

Die Reinheit des Extraktes wird mittels Gaschromatographie (GC) überprüft. Die Ergebnisse sind in der Übersicht gemäß Anlage 1 dargestellt.

Folgende Werte wurden gemessen:
- Betulin:: minimal 80 %
- Betulinsäure:: maximal 10 %
- Lupeol:: maximal 3 %
- Oleanolsäure:: maximal 4 %

Die Übersicht zeigt ein typisches Chromatogramm mit Werten von 84 bis 86 % Betulin, 4 bis 5 % Betulinsäure, ca. 1 % Lupeol und ca. 1 % Oleanolsäure.

Gemäß den Bestimmungen der ICH muß die restliche Menge des Lösungsmittels n-Hexan < 7,250 mg/kg Extrakt betragen, weil eine Creme enthaltend 4% Birkenkorkextrakt nicht mehr als 290 mg/kg n-Hexan aufweisen darf. Typische Werte betragen 1,500 mg/kg n-Hexan im trockenen Extrakt, was ca. 60 mg/kg in der Birkencreme entspricht.

### Endkontrolle:

Jede Charge wird gemäß den europäischen Bestimmungen hinsichtlich der Säurewerte (< 5) und der Zahl der Peroxide (< 15) mittels eines standardisierten Verfahrens untersucht. Der Gehalt an Birkenkorkextrakt wird mittels GC analysiert. Es erfolgt weiterhin eine Untersuchung bezüglich Keimen (< 100/g), außerdem dürfen keine pathogenen Keime enthalten sein.

### III. Erfindungsgemäße Formulierungen (Birkencreme):

Die erfindungsgemäßen Formulierungen sind in der Tabelle 1 (Tab.1) zusammengefaßt. Sie weisen folgende Zusammensetzung auf:

**Tab. 1:**

| Zusammensetzungen der Formulierungen | | |
|---|---|---|
| Aktiver Bestandteil in allen Formulierungen | Extrakt aus Birkenkork 40 ± 4 mg/g Creme (4 %) | |
| Formulierung | Bestandteile in in % | |
| Birkencreme A | 29.3 | Avocadoöl |
| | 14.7 | Mandelöl |
| | 52 | Wasser |
| Birkencreme G | 29.3 | Avocadoöl |
| | 14.7 | Mandelöl |
| | 5 | Glycerin |
| | 47 | Wasser |
| Birkencreme H | 29.3 | Avocadoöl |
| | 14.7 | Mandelöl |
| | 5 | Harnstoff |
| | 47 | Wasser |

Alle drei Formulierungen enthalten dieselbe Menge an aktivem Bestandteil (Birkenkorkextrakt) sowie Avocado- und Mandelöl. Sie unterscheiden sich lediglich hinsichtlich der Bestandteile Harnstoff (nur in der Birkencreme H) sowie Glycerin (nur in der Birkencreme G), Birkencreme A ist frei von diesen beiden Bestandteilen.

### Stabilitätstest:

Die Birkencreme kann bei 500 x g zentrifugiert werden und ist dabei stabil.

### IV. Toxikologische Untersuchungen:

### 1. Akute Toxizität

Die akute Toxizität des Birkenkorkextraktes wurde durch intraperitoneale und subkutane Verabreichung an Mäusen und Ratten bestimmt.

### Mäuse:

Einzelne Dosen des Birkenkorkextraktes (Chargen-Nummer: Bet. 001) in Methocel als Trägerstoff wurden fünf männlichen und fünf weiblichen CD-1 Mäusen subkutan verabreicht. Die Tiere wurden mit einer Dosis von 2000 mg/kg bezogen auf das Körpergewicht behandelt.

Die Sterblichkeitsrate wurde 14 Tage nach der Gabe einer einzelnen Dosis zur Festlegung der durchschnittlichen letalen Dosis LD₅₀ bestimmt. Im gleichen Zeitraum wurden die Tiere beobachtet, um Anzeichen einer Toxizität festzustellen. Als solche kommen lokale und systemische Intoleranz-Reaktionen sowie Veränderungen des Körpergewichts in Frage. Die Tiere wurden am Ende des Versuchs zeitraums geopfert und makroskopisch untersucht. Der akute Toxizitätstest beruhte auf der EC Richtlinie L 383 A: B1.

Unter den gegebenen Testbedingungen (2000 mg Birkenkorkextrakt/kg Körpergewicht) wurden keine Intoleranzreaktionen beobachtet. Die Tiere zeigten während des Versuchszeitraums die erwartete Gewichtszunahme. Die Ergebnisse sind in Tabelle 2 zusammengefaßt.

**Tab. 2:**

| Akute Toxizität in Mäusen Durchschnittliches Körpergewicht während des Versuchszeitraums | | |
|---|---|---|
| | Durchschnittl. Körpergewicht(g) | |
| | Männlich | weiblich |
| Beginn | 22.8 | 19.8 |
| Tag 7 | 27.0 | 22.2 |
| Tag 14 | 31.2 | 23.6 |

Bei der makroskopischen Untersuchung wurden in drei männlichen und drei weiblichen Tieren lediglich Veränderungen festgestellt, die auf das angewandte technische Untersuchungsverfahren zurückzuführen sind.

In diesem Toxizitätstest liegen der Bereich der ersten Intoleranzreaktion, der niedrigste letale Wert sowie der LD₅₀ Wert über 2000 mg Birkenkorkextrakt/kg Körpergewicht.

Bei intraperitonealer Verabreichung des Birkenkorkextraktes an Mäuse wurden toxische Symptome oberhalb von 500 mg/kg Körpergewicht beobachtet. Es wurden nur leichte toxische Reaktionen, wie reduziertes passives Bewegungsvermögen, Bewegungskoordinationsstörungen und reduzierter Muskeldehnungswiderstand beobachtet. Hieraus wurde geschlossen, daß die toxischen Symptome auf eine Bauchfellentzündung (Peritonitis) zurückzuführen sind, die durch die Extraktlösung hervorgerufen wurde. Ein LD₅₀ Wert konnte nicht festgestellt werden, so daß er über den Beobachtungszeitraum vom 14 Tagen oberhalb von 2000 mg/kg Körpergewicht liegt.

### Ratten:

Einzelne Dosen des Birkenkorkextraktes (Chargen-Nummer: Bet. 001) in Methocel als Trägerstoff wurden fünf männlichen und fünf weiblichen Sprague-Dawley Ratten subkutan verabreicht. Die Tiere wurden mit einer Dosis von 2000 mg/kg bezogen auf das Körpergewicht behandelt.

Die Sterblichkeitsrate und die toxischen Symptome wurden 14 Tage nach der Gabe einer einzelnen Dosis bestimmt. Die Tiere wurden am Ende des Versuchszeitraums geopfert und makroskopisch untersucht. Der akute Toxizitätstest beruhte auf der EC Richtlinie L 383 A: B1.

Unter den gegebenen Testbedingungen (2000 mg Birkenkorkextrakt/kg Körpergewicht) wurden keine Intoleranzreaktion oder Sterblichkeit beobachtet. Die Tiere zeigten während des Versuchszeitraums die erwartete Gewichtszunahme.

Bei der makroskopischen Untersuchung wurden in einem männlichen und drei weiblichen Tieren lediglich Veränderungen festgestellt, die auf das angewandte technische Untersuchungsverfahren zurückzuführen sind. Die Ergebnisse sind in Tabelle 3 (Tab.3) zusammengefasst.

**Tab. 3:**

| Akute Toxizität in Ratten | | |
|---|---|---|
| Symptome/Kriterien | Sprague-Dawley Ratten (n=5/Geschlecht) Birkenkorkextrakt 2000 mg/kg Körpergewicht | |
| | männlich | weiblich |
| Lokale Intoleranzreaktionen | Keine | keine |
| Systemische Intoleranzreaktionen | Keine | keine |
| Sterblichkeit innerhalb von 6/24 h in 7/14 Tagen | Keine | keine |
| Durchschnittl. Körpergewicht (g) | | |
| Beginn | 187.6 | 174.4 |
| Tag 7 | 239.8 | 203.8 |
| Tag 14 | 280.8 | 218.0 |
| Hemmung des Körpergewichts | keine | keine |
| Makroskopischer Befund | 1/5 | 3/5 |

In diesem Toxizitätstest liegen der Bereich der ersten Intoleranzreaktionen, der niedrigste letale Wert sowie der LD₅₀ Wert über 2000 mg Birkenkorkextrakt/kg Körpergewicht.

Bei intraperitonealer Verabreichung des Birkenkorkextraktes an Ratten wurden toxische Symptome oberhalb von 500 mg/kg Körpergewicht beobachtet. Es wurden nur leichte toxische Reaktionen wie reduziertes passives Bewegungsvermögen, Bewegungskoordinationsstörungen und reduzierter Muskeldehnungswiderstand beobachtet. Hieraus wurde geschlossen, daß die toxischen Symptome auf eine Bauchfellentzündung (Peritonitis) zurückzuführen sind, die durch die Extraktlösung hervorgerufen wurde. Ein LD₅₀ Wert konnte nicht festgestellt werden, so daß er über den Beobachtungszeitraum vom 14 Tagen oberhalb von 2000 mg/kg Körpergewicht liegt.

### Schlußfolgerung:

Die OECD Richtlinien legen fest, daß keine weiteren Versuche nötig sind, wenn über einen Zeitraum von 14 Tagen bei einer Verabreichung von 2000 mg/kg Körpergewicht an Mäuse und Ratten (je fünf Tiere/Geschlecht) keine durch das getestete Produkt begründete Sterblichkeit vorliegt.

Chemikalien werden nach ihrer relativen Toxizität eingeordnet. Sie werden als toxisch bezeichnet, wenn der LD₅₀ Wert zwischen 50 und 500 mg/kg Körpergewicht liegt. Bei einem LD₅₀ Wert von 5000 bis 15000 mg/kg Körpergewicht wird praktisch von keiner Toxizität ausgegangen.

Im vorliegenden Fall wird von einem LD₅₀ Wert > 2000 mg/kg Körpergewicht ausgegangen wird, der wahrscheinlich auch > 5000 mg/kg Körpergewicht liegt, weil selbst bei dem hohen Wert von 2000 mg/kg Körpergewicht keine toxischen Symptome beobachtet wurden. Wie aus den Versuchsergebnissen ersichtlich ist, kann der Birkenkorkextrakt als nicht toxisch bezeichnet werden.

### 2. Subakute Toxizität

### Ratten, intraperitoneal (i.p.)

Birkenkorkextrakt (Chargen-Nummer: Bet. 009) wurde Versuchsratten (Sprague-Dawley/Crl: CD® BR) mittels intraperitonealer Injektion über 14 Tage im Rahmen einer subakuten Toxizitätsuntersuchung verabreicht. Das Versuchsprotokoll ist in Tabelle 4 (Tab.4) zusammengefaßt.

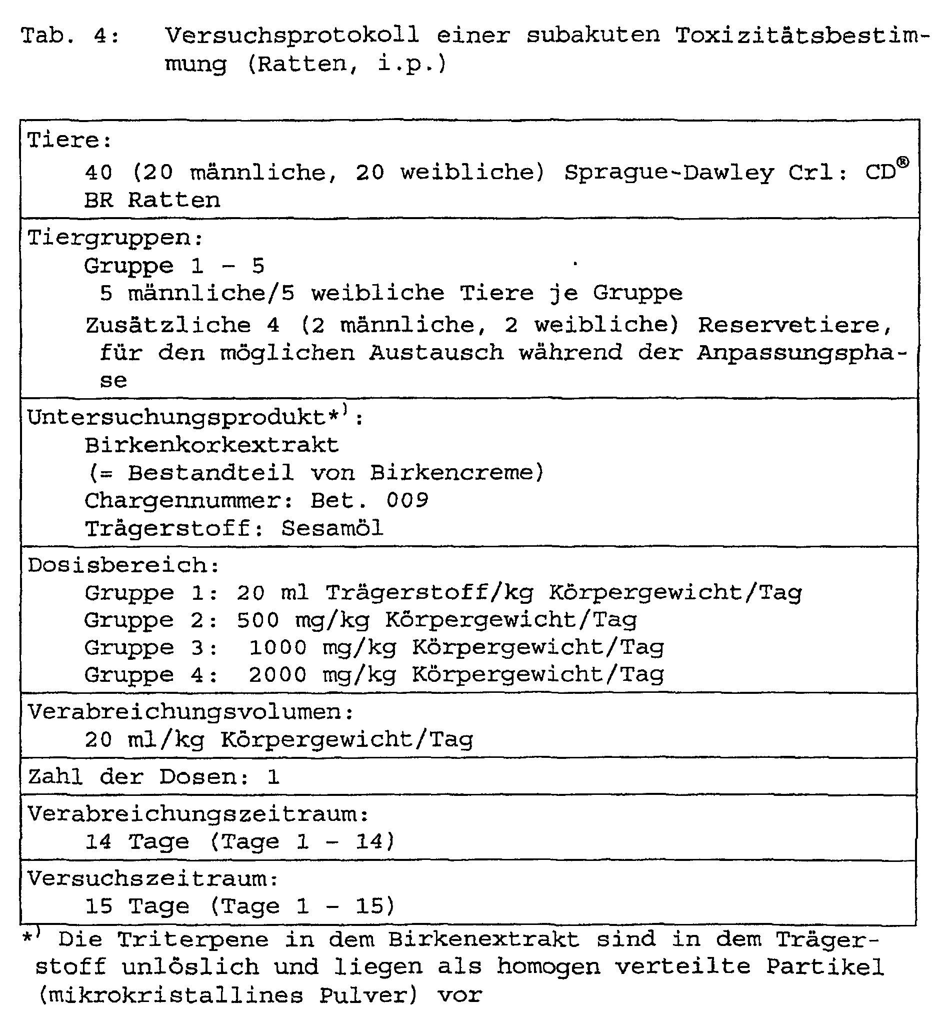

Diese subakute Toxizitätsuntersuchung wurde für die Ermittlung der Dosis verwendet, die in einer vierwöchigen subchronischen Toxizitätsuntersuchung eingesetzt wurde. Die Ergebnisse der Toxizitätsuntersuchung sind in Tabelle 5 (Tab.5) gezeigt.

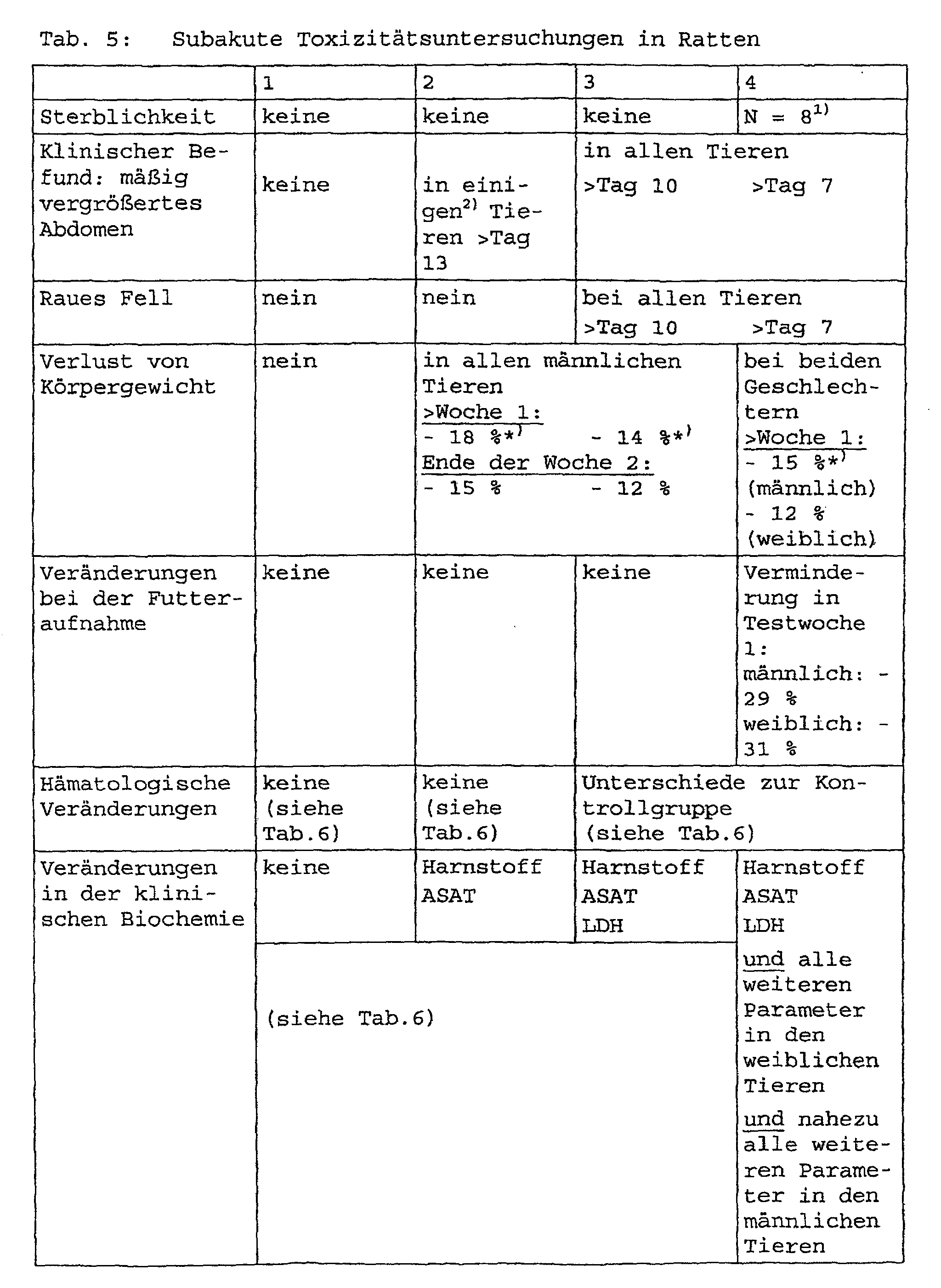

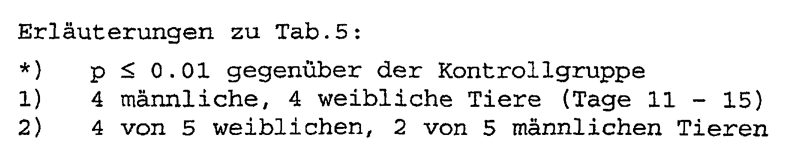

Die Veränderungen in der klinischen Biochemie (Tab.5) sind nur gegenüber der Kontrollgruppe in den Gruppen 3 und 4 signifikant (p ≤ 0.01); in Gruppe 3 hinsichtlich der ASAT Aktivität (+ 113/+132 %), in Gruppe 4 nur bei weiblichen Tieren hinsichtlich der Parameter: Gesamtprotein, Harnstoff im Blut, Calcium, Kalium und LDH Aktivität.

**Tab. 6:**

| Hämatologische Veränderungen in der subakuten Toxizitätsuntersuchung (Ratten, i.p.) - Definitionen der Gruppen 3 und 4 vgl. Tab.5 | | | | |
|---|---|---|---|---|
| Parameter | Gruppe | | | |
| | 3 Veränderung in % | | 4 Veränderung in % | |
| | männlich | weiblich | männlich | weiblich |
| Plättchenzählung | | + 47**⁾ | + 80 | + 54**⁾ |
| Retikulozytenzählung | - | - | + 64 | + 36**⁾ |
| Leukozytenzählung | - | - | - | - 34 |

| | | | | |
|---|---|---|---|---|
| Erläuterungen zu Tab.6: **) p ≤ 0.01 gegenüber der Kontrollgruppe | | | | |

Die äußerliche Untersuchung vor der nekroskopischen Untersuchung zeigte eine mäßige bis schwere Vergrößerung des Abdomens in allen Gruppen, mit Ausnahme der Kontrollgruppe, in der das Abdomen bei allen Tieren nur gering vergrößert war. Die Ergebnisse der makroskopischen post mortem Untersuchung sind in Tabelle 7 (Tab.7) gezeigt.

**Tab. 7:**

| Nekroskopischer Befund in der subakuten Toxizitätsuntersuchung - Definitionen der Gruppen vgl. Tab.4 | | | | |
|---|---|---|---|---|
| | Gruppe | | | |
| | 1 | 2 | 3 | 4 |
| Weiß-gelbliche Flüssigkeit im abdominalen Raum¹⁾ | + | + | + | + |
| Weiße Ablagerungen oder Schichten auf der Scheidewand (Zwerchfell) oder auf den abdominalen Organen | keine | + | + | + |
| Bauchfellentzündung | keine | keine | keine | + |
| Abdominaler Raum gefüllt mit weißen pulpartigen/ öligen Inhalten | nein | nein | nein | + ca. 30 ml |

| | | | | |
|---|---|---|---|---|
| Erläuterungen zu Tab.7: ¹⁾ in 4 Tieren | | | | |

In dieser zweiwöchigen Untersuchung zur Bestimmung des Dosisbereichs lag der Bereich ohne Effekt < 500 mg Birkenextrakt/kg Körpergewicht. Mäßig bis stark vergrößerte Abdomen wurden bei 500 mg/kg Körpergewicht und höher beobachtet. Signifikante Reduktionen des Körpergewichts wurden in allen behandelten Gruppen festgestellt, jedoch nur bei den männlichen Tieren. Raues Fell wurde bei 1000 mg/kg Körpergewicht und höher beobachtet. Einige hämatologische und/oder biochemische Parameter waren signifikant bei 1000 mg/kg Körpergewicht und höher verändert. Die nekroskopische Untersuchung zeigte weißliche Ablagerungen auf dem Zwerchfell oder auf den abdominalen Organen, die teilweise von Adhäsionen begleitet wurden bei 500 mg/kg Körpergewicht und höher. Bei allen Gruppen war der abdominale Raum mit einer öligen/wäßrigen Flüssigkeit gefüllt, was auf den Trägerstoff Sesamöl zurückzuführen ist. Bei 2000 mg/kg Körpergewicht starben 8 von 10 Tieren. Die nekroskopische Untersuchung ergab: 1) Bauchfellentzündung in 4 der vorzeitig verstorbenen Tiere; 2) in allen Tieren war der abdominale Raum mit pulpartigen/öligen Inhalten gefüllt. Für die subchronische Toxizitätsuntersuchung wurde ein Bereich von 60 bis 540 mg/kg Körpergewicht ausgewählt.

### Hunde (Beagle), i.p.

Eine subakute Toxizitätsuntersuchung wurde über zwei Wochen mit Beagle als Dosisbestimmungs-Versuch über 4 Wochen als subchronische Toxizitätsuntersuchung durchgeführt. Der Birkenkorkextrakt (Chargennummer: Bet. 009) wurde mit Sesamöl als Trägerstoff intraperitoneal verabreicht. Die Ergebnisse sind in Tabelle 8 (Tab.8) zusammengefaßt.

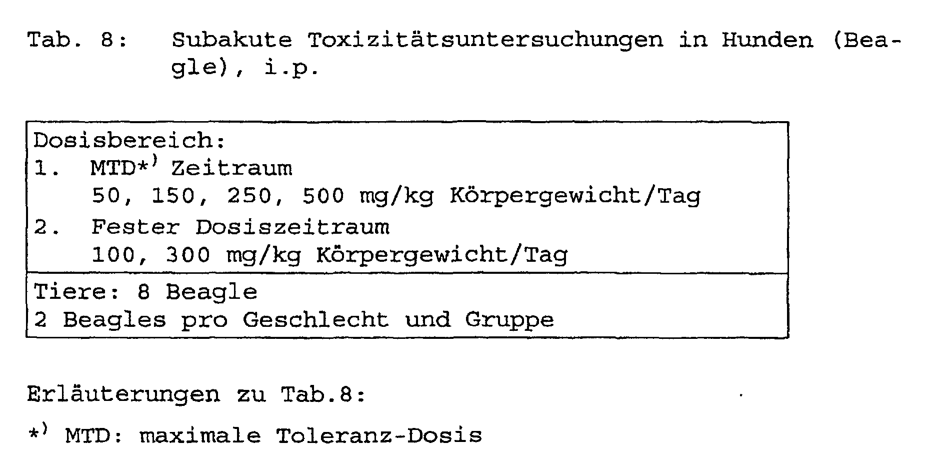

Die Ergebnisse sind in Tabelle 9 (Tab. 9) gezeigt.

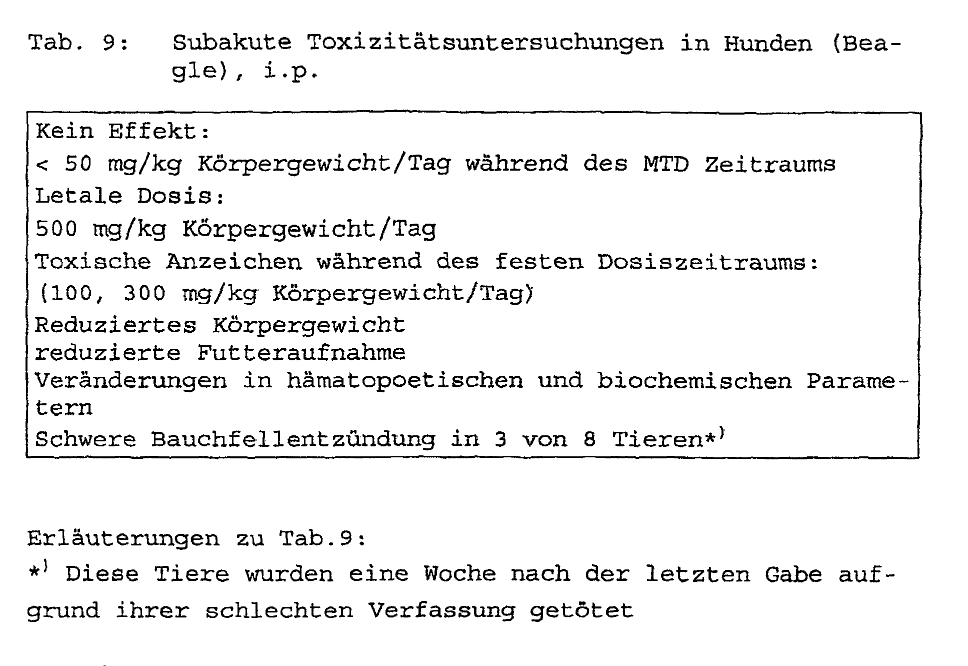

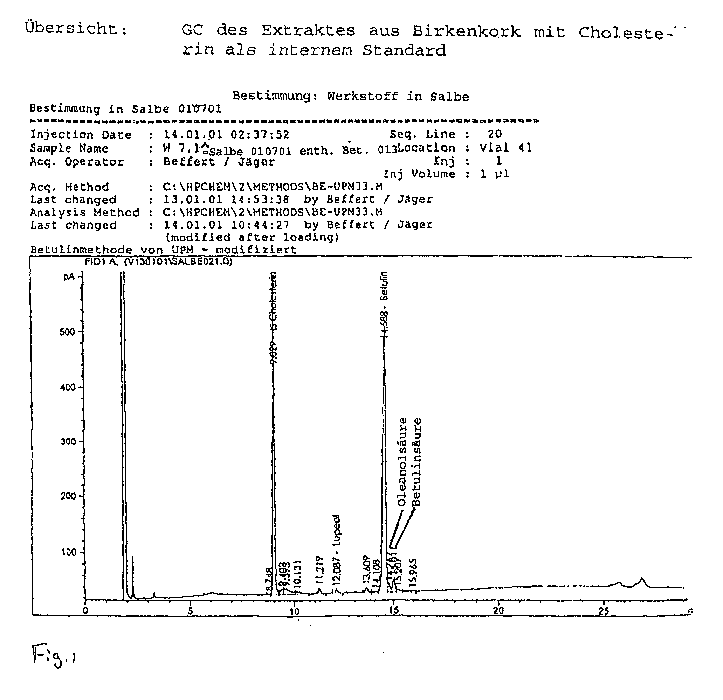

## Patentansprüche

1. Verfahren zur kontinuierlichen Gewinnung von Triterpenen aus Pflanzen und/oder deren Bestandteilen, das die Schritte umfaßt:
(a) kontinuierliches Zuführen von Pflanzenteilen und eines Lösungsmittels, in dem die Triterpene nicht oder nur gering löslich sind, und Waschen der Pflanzenteile mit dem Lösungsmittel bei 20°C bis 70°C im Gegenstrom;
(b) kontinuierliches Extrahieren der Triterpene mit einem Lösungsmittel unter erhöhtem Druck und erhöhter Temperatur im Gegenstrom;
(c) kontinuierliches Abkühlen und Entspannen der die Triterpene enthaltenden Lösung, wodurch die Triterpene aus dem Lösungsmittel aus Schritt (b) auskristallisieren;
(d) Filtrieren der Triterpene bei Raumtemperatur; und
(e) Waschen der Triterpene mit einem Lösungsmittel.

2. Verfahren nach Anspruch 1, **dadurch gekennzeichnet, daß** die Triterpene aus Birkenrinde, vorzugsweise aus dem weißen Teil der Birkenrinde (Birkenkork) gewonnen werden.

3. Verfahren nach einem der Ansprüche 1 oder 2, **dadurch gekennzeichnet, daß** die Triterpene in einem Reinheitsgrad von mindestens 80 %, vorzugsweise 85 %, insbesondere 90 %, besonders bevorzugt von über 90 % gewonnen werden.

4. verfahren nach einem der Ansprüche 1 bis 3, **dadurch gekennzeichnet, daß** der Hauptbestandteil der extrahierten Triterpene Betulin ist.

5. Verfahren nach einem der Ansprüche 1 bis 4, **dadurch gekennzeichnet, daß** der Gehalt an Betulin mindestens 80 %, vorzugsweise 85 %, insbesondere 90 %, besonders bevorzugt über 90 % beträgt.

6. Verfahren nach einem der Ansprüche 1 bis 5, **dadurch gekennzeichnet, daß** in den Schritten (a), (b) und (e) das gleiche Lösungsmittel verwendet wird.

7. Verfahren nach einem der Ansprüche 1 bis 6, **dadurch gekennzeichnet, daß** als Lösungsmittel überkritisches CO₂, ein niedrig siedender Kohlenwasserstoff oder ein Gemisch, das einen niedrig siedenden Kohlenwasserstoff aufweist, verwendet wird.

8. Verfahren nach einem der Ansprüche 1 bis 7, **dadurch gekennzeichnet**-, daß als Lösungsmittel n-Pentan, n-Hexan oder n-Heptan verwendet wird.

9. Verfahren nach einem der Ansprüche 1 bis 8, **dadurch gekennzeichnet, daß** für Schritt (a) das Lösungsmittel verwendet wird, das nach Schritt (b) anfällt.

10. Verfahren nach einem der Ansprüche 1 bis 9, **dadurch gekennzeichnet, daß** das Waschen in Schritt (a) unter einem Druck von 1 bis 300 bar, vorzugsweise 10 bis 35 bar, besonders bevorzugt 25 bar, erfolgt.

11. Verfahren nach einem der Ansprüche 1 bis 10, **dadurch gekennzeichnet, daß** das Extrahieren in Schritt (b) bei einer Temperatur von 50 bis 200°C, vorzugsweise 140 bis 160°C, besonders bevorzugt 150°C und unter einem Druck von 10 bis 300 bar, vorzugsweise 1 bis 35 bar, besonders bevorzugt 25 bar, erfolgt.

12. Verfahren nach einem der Ansprüche 1 bis 11, **dadurch gekennzeichnet, daß** das Auskristallisieren in Schritt (c) als Mikrokristallisation mit einer durchschnittlichen Korngröße von < 40 µm, insbesondere von 2 bis 32 µm erfolgt.

13. Emulsion deren wäßrige und fettige Phase durch einen Pflanzenextrakt emulgiert wird, wobei der Pflanzenextrakt wenigstens ein Triterpen und/oder wenigstens ein Derivat eines Triterpens und die Emulsion weiterhin wenigstens ein Öl und/oder Fett und Wasser aufweist, **dadurch gekennzeichnet, daß** das Triterpen und/oder sein Derivat die Emulsion emulgiert, pharmazeutisch aktiv ist und als einziger Konservierungsstoff vorhanden ist.

14. Emulsion nach Anspruch 13, **dadurch gekennzeichnet, daß** der Pflanzenextrakt ein Extrakt aus der Rinde von Birken, vorzugsweise aus dem weißen Teil der Birkenrinde (Birkenkork) ist.

15. Emulsion nach Anspruch 13 oder 14, **dadurch gekennzeichnet, daß** der Pflanzenextrakt wenigstens eine der Substanzen Betulin, Betulinsäure, Lupeol, Erythrodiol, Allobetulin, Phellonsäure, Hydroxylakton, Betulinaldehyd, β-Amyrin, Oleanolsäure, Ursolsäure, verestertes Betulin und/oder β-Sitosterol aufweist.

16. Emulsion nach einem der Ansprüche 13 bis 15, **dadurch gekennzeichnet, daß** der Anteil des Triterpens und/oder seines Derivates 2 bis 10 % am Gesamtgewicht der Emulsion beträgt.

17. Emulsion nach Anspruch 16, **dadurch gekennzeichnet, daß** der Anteil des Triterpens und/oder seines Derivates 2 bis 3,5 % oder 3,5 bis 10 % am Gesamtgewicht der Emulsion beträgt.

18. Emulsion nach einem der Ansprüche 13 bis 17, **dadurch gekennzeichnet, daß** das Öl Avocado- und/oder Mandelöl ist.

19. Emulsion nach einem der Ansprüche 13 bis 18, **dadurch gekennzeichnet, daß** die Emulsion weiterhin wenigstens ein Feuchthaltemittel und/oder wenigstens ein Verdickungsmittel aufweist.

20. Emulsion nach Anspruch 19, **dadurch gekennzeichnet, daß** das Feuchthaltemittel Glycerin und/oder Harnstoff ist, das in einer Konzentration von 3 bis 10 %, vorzugsweise von je 5 %, bezogen auf die Menge der Emulsion, zugesetzt ist.

21. Emulsion nach Anspruch 19, **dadurch gekennzeichnet, daß** das Verdickuugsmittel ein Polysaccharid ist, das in einer Konzentration von 0,2 bis 2 %, vorzugsweise 0,5 %, bezogen auf die Menge des Wassers, zugesetzt ist.

22. Emulsion nach Anspruch 21, **dadurch gekennzeichnet, daß** das polysaccharid Agar Agar oder Carrageenan ist.

23. Emulsion nach einem der Ansprüche 13 bis 22, **dadurch gekennzeichnet, daß** die Emulsion 2 bis 10 % Extrakt aus dem weißen Teil der Birkenrinde, 20 bis 30 % Avocadoöl, 10 bis 20 % Mandelöl und 40 bis 68 % Wasser aufweist.

24. Emulsion nach Anspruch 23, **dadurch gekennzeichnet, daß** die Emulsion 4 % Extrakt aus dem weißen Teil der Birkenrinde, 29,3 % Avocadoöl, 14,7 % Mandelöl und 52 % Wasser aufweist.

25. Emulsion nach einem der Ansprüche 13 bis 22, **dadurch gekennzeichnet, daß** die Emulsion 2 bis 10 % Extrakt aus dem weißen Teil der Birkenrinde, 20 bis 30 % Avocadoöl, 10 bis 20 % Mandelöl, 5 bis 10 % Feuchthaltemittel und 30 bis 63 % Wasser aufweist.

26. Emulsion nach Anspruch 25, **dadurch gekennzeichnet, daß** die Emulsion 4 % Extrakt aus dem weißen Teil der Birkenrinde, 29,3 % Avocadoöl, 14,7 % Mandelöl, 5 % Glycerin und/oder 5 % Harnstoff und 42 bis 47 % Wasser aufweist.

27. Emulsion nach einem der Ansprüche 13 bis 26, **dadurch gekennzeichnet, daß** der Extrakt aus dem weißen Teil der Birkenrinde mindestens 80 % Betulin, maximal 10 % Betulinsäure, maximal 3 % Lupeol und maximal 4 % oleanolsäure aufweist.

28. Verfahren zur Herstellung einer Emulsion nach einem der Ansprüche 13 bis 27, das die Schritte aufweist:
(a) Dispergieren des Triterpens in einem Öl und/oder Fett;
(b) Emulgieren von Wasser;
(c) Homogenisieren der Emulsion bis zur erforderlichen Konsistenz.

29. Verfahren nach Anspruch 28, **dadurch gekennzeichnet, daß** das Dispergieren und das Homogenisieren in einem Homogenisiermischer erfolgt.

30. Verfahren nach Anspruch 28 oder 29, **dadurch gekennzeichnet, daß** dem Wasser in Schritt (b) ein Feuchthaltemittel, insbesondere Glycerin und/oder Harnstoff, oder ein Verdickungsmittel, insbesondere ein Polysaccharid zugesetzt ist.

31. Verwendung der Emulsion nach einem der Ansprüche 13-27 zur Herstellung eines Kosmetikums, vorzugsweise in Form einer Salbe, einer Lotion, einer Creme, eines Gels, eines Gelees oder eines Shampoos, insbesondere zum Auftragen auf die Haut, die Kopfhaut und/oder zum Inhalieren.

32. Verwendung der Emulsion nach einem der Ansprüche 13-27 zur Herstellung eines Arzneimittels, vorzugsweise in Form einer Salbe, einer Lotion, einer Creme, eines Gels, eines Gelees oder eines Shampoos, insbesondere zum Auftragen auf die Haut, die Kopfhaut und/oder zum Inhalieren, besonders bevorzugt bei dermatologischen Veränderungen der Haut und/oder der Kopfhaut, vor allem bei Neurodermitis, Psoriasis, seborrhoischem Ekzem, Melanodermie, Präcancerosen der Haut sowie zum Inhalieren bei asthmatischen Anfällen und/oder zur Substitution von Glucocorticoiden.

## Claims

1. A continuous process for obtaining triterpenes from plants and/or their components, comprising the following steps:
(a) continuously introducing plant parts and a solvent, in which solvent the triterpenes are not soluble or only slightly soluble, and washing the plant parts with said solvent at 20°C to 70°C in counter-current;
(b) continuously extracting triterpenes with a solvent under increased pressure and elevated temperature in counter-current;
(c) continuously cooling and depressurizing the triterpene containing solution, whereby triterpene crystallizes out of the solvent of step (b);
(d) filtering the triterpenes at room temperature;
(e) washing the triterpenes in a solvent.

2. A process according to Claim 1, **characterized in that** the triterpenes are obtained from birch bark, preferably from the white part of the birch bark (birch cork).

3. A process according to Claim 1 or 2, **characterized in that** the triterpenes are obtained at a purity of at least 80%, preferably 80%, in particular 90%, and particularly preferrably above 90%.

4. A process according to one of the claims 1 to 3, **characterized in that** the main component of the extracted triterpene is betulin.

5. A process according to one of the claims 1 to 4, **characterized in that** the content of betulin is at least 80% preferably 85%, in particular 90%, particularly preferably above 90%.

6. A process according to one of the claims 1 to 5, **characterized in that** the same solvent is employed in steps (a), (b) and (e).

7. A process according to one of the claims 1 to 6, **characterized in that** the solvent is selected from the group consisting of supercritical CO₂, a low boiling carbohydrate or a mixture which includes a low boiling carbohydrate.

8. A process according to one of the claims 1 to 7, **characterized in that** the solvent is selected from the group consisting of n-pentane, n-hexane and n-heptane.

9. A process according to one of the claims 1 to 8, **characterized in that** the solvent used for step (a) is the solvent which is collected following step (b).

10. A process according to one of the claims 1 to 9, **characterized in that** the washing in step (a) occurs under a pressure of 1 to 300 bar, preferably 10 to 35 bar, particularly preferably 25 bar.

11. A process according to one of the claims 1 to 12, **characterized in that** the extraction in step (b) occurs at a temperature of 50 to 200°C, preferably 140 to 160°C, particularly preferably at about 150°C and under a pressure of 1 to 300 bar, preferably 10 to 35 bar, particularly preferably about 25 bar.

12. A process according to one of the claims 1 to 11, **characterized in that** the crystallization out in step (c) occurs as micro-crystallization with an average particle size of < 40 µm, in particular from 2 to 32 µm.

13. An emulsion wherein the aqueous and fatty phases are emulsified using a plant extract, wherein the plant extract is at least one triterpene and/or at least one derivate of a triterpene and the emulsion further contains at least one oil and/or fat and water, **characterized in that** the triterpene and/or it derivative is a preservative, emulsifier and pharmaceutical active substance in the emulsion.

14. An emulsion according to Claim 13, **characterized in that** the plant extract is an extract from the bark of birch, preferably from the white part of the birch bark (birch cork).

15. An emulsion according to Claim 13 or 14, **characterized in that** the plant extract is at least one substance selected from the group consisting of betulin, betulinic acid, lupeol, erythrodiol, allobetulin, phellonic acid, hydroxy-lactone, betulin aldyhyde, β-amyrin, oleanolic acid, ursolic acid, esterified betulin and/or β-sitosteol.

16. An emulsion according to one of the claims 13 to 15, **characterized in that** the proportion of triterpene and/or its derivative comprises 2 to 10% of the total weight of the emulsion.

17. An emulsion according to Claim 16, **characterized in that** the proportion of triterpene and/or it derivative comprises 2 to 3.5% or 3.5 to 10% of the total weight of the emulsion.

18. An emulsion according to one of the claims 13 to 17, **characterized in that** the oil is avocado oil and/or almond oil.

19. An emulsion according to one of the claims 13 to 18, **characterized in that** the emulsion further includes at least one moisturizing agent and/or at least one thickening agent.

20. An emulsion according to Claim 19, **characterized in that** the moisturizing agent is glycerin and/or urea, which is added in a concentration of 3 to 10%, preferably 5% of each, based upon the amount of the emulsion.

21. An emulsion according to Claim 19, **characterized in that** the thickening agent is a polysaccharide, which is added in a concentration of 0.2 to 2%, preferably 0.5%, based upon the amount of water.

22. An emulsion according to Claim 21, wherein the polysaccharide is agar agar or carrageenan.

23. An emulsion according to one of the claims 13 to 22, **characterized in that** the emulsion includes 2 to 10% extract from the white part of the birch bark, 20 to 30% avocado oil, 10 to 20% almond oil and 40 to 68% water.

24. An emulsion according to Claim 23, **characterized in that** the emulsion comprises 4% extract from the white part of the birch bark, 29.3% avocado oil, 14.7% almond oil and 52% water.

25. An emulsion according to one of the claims 13 to 22, **characterized in that** the emulsion comprises 2 to 10% extract from the white part of the birch bark, 20 to 30% avocado oil, 10 to 20% almond oil, 5 to 10% moisturizing agent and 30 to 63% water.

26. An emulsion according to Claim 25, **characterized in that** the emulsion comprises 4% extract from the white part of the birch bark, 29.3% avocado oil, 14.7% almond oil, 5% glycerin and/or 5% urea and from 42 to 47% water.

27. An emulsion according to one of the claims 13 to 26, **characterized in that** the extract from the white part of the birch bark comprises at least 80% betulin, at most 10% betulinic acid, at most 3% lupeol and at most 4% oleanolic acid.

28. A process for producing an emulsion according to one of the claims 13 to 27 which comprises the steps:
(a) dispersing the triterpene in an oil and/or fat;
(b) emulsifying water;
(c) homogenizing the emulsion to the desired consistency.

29. A process according to Claim 28, **characterized in that** the dispersing and the homogenizing steps are carried out in a homogenizer mixer.

30. A process according to Claim 28 or 29, wherein a moisturizing agent is added to the water in step (b), in particular glycerin and/or urea, or a thickening agent, in particular a polysaccharide.

31. Use of the emulsion according to claims 13 to 27 for manufacture of a cosmetic, preferably in the form of a salve, a lotion, a cream, a gel, a jelly, or a shampoo, in particular for application to the skin, the face or scalp and/or for inhalation.

32. Use of the emulsion according to claims 13 to 27 for manufacture of a medicament, preferably in the form of a salve, a lotion, a cream, a gel, a jelly, or a shampoo, in particular for application to the skin, the face or scalp and/or for inhalation, particularly preferably for dermatological conditions of the skin and/or the scalp, above all for neuodermitis, psoriasis, seborrheic eczema, melanoderma, pre-cancerous conditions of the skin as well as inhalation in asthmatic attacks and/or for substitution in place of glucocorticoids.

## Revendications

1. Procédé pour extraire en continu des triterpènes contenus dans des plantes et/ou des composants de ces plantes, présentant les étapes suivantes :
a) Amenée en continu de parties de plantes et d'un solvant dans lequel les triterpènes sont peu ou pas solubles, et lavage des parties des plantes par le solvant à contre-courant, entre 20° et 70° C.
b) Extraction continue des triterpènes par un solvant sous pression élevée et à haute température.
c) Refroidissement continu et détente de la solution contenant les triterpènes de sorte que ceux-ci cristallisent en se séparant des solvants de la phase b)
d) Filtration des triterpènes à la température ambiante.
e) Lavage des triterpènes au moyen d'un solvant.

2. Procédé selon la revendication 1,
**caractérisé en ce que**
les triterpènes sont extraits d'écorces du bouleau, de préférence de la partie blanche de l'écorce (suber de bouleau).

3. Procédé selon l'une des revendications 1 ou 2,
**caractérisé en ce que**
les triterpènes sont extraits avec un degré de pureté d'au moins 40 %, avantageusement 85 %, notamment 90 % et tout à fait préférentiellement de plus de 90 %.

4. Procédé selon l'une des revendications 1 à 3,
**caractérisé en ce que**
le composant principal des triterpènes extraits est la bétuline.

5. Procédé selon l'une des revendications 1 à 4,
**caractérisé en ce que**
la teneur en bétuline est d'au moins 80 %, avantageusement 85 %, notamment 90 % et tout à fait préférentiellement de plus de 90 %.

6. Procédé selon l'une des revendications 1 à 5,
**caractérisé en ce que**
le même solvant est utilisé dans les étapes (a), (b), et (c).

7. Procédé selon l'une des revendications 1 à 6,
**caractérisé en ce que**
le solvant utilisé est du CO₂ hypercritique, un hydrocarbure à bas point d'ébullition ou un mélange contenant un tel hydrocarbure.

8. Procédé selon l'une des revendications 1 à 7,
**caractérisé en ce que**
le solvant utilisé est du n-pentane, du n-hexane ou du n-heptane.

9. Procédé selon l'une des revendications 1 à 8,
**caractérisé en ce que**
pour l'étape (a), le solvant utilisé est celui qui est produit après l'étape (b).

10. Procédé selon l'une des revendications 1 à 9,
**caractérisé en ce que**
le lavage à l'étape (a) a lieu sous une pression de 1 à 300 bars, de préférence 10 à 35 bars, très préférentiellement à 25 bars.

11. Procédé selon l'une des revendications 1 à 10,
**caractérisé en ce que**
l'extraction à l'étape (b) a lieu à une température de 50 à 200° C, de préférence 140 à 200° C, très préférentiellement à 150° C et sous une pression de 10 à 300 bars, de préférence 10 à 35 bars, très préférentiellement à 25 bars.

12. Procédé selon l'une des revendications 1 à 11,
**caractérisé en ce que**
la cristallisation à l'étape (c) est une micro cristallisation avec une dimension de cristaux inférieure à 40 µm, en particulier de 2 à 32 µm.

13. Emulsion en phase aqueuse et huileuse d'un extrait de plantes, cet extrait contenant au moins un triterpène et/ou au moins un dérivé d'un triterpène, l'émulsion contenant au moins de l'huile et/ou de la graisse et de l'eau
**caractérisé en ce que**
la triterpène et/ou son dérivé produit l'émulsion, est actif sur le plan pharmaceutique et est présent en tant que seul élément de conservation.

14. Emulsion selon la revendication 13,
**caractérisée en ce que**
l'extrait de plantes est un extrait d'écorce de bouleau, de préférence de la partie blanche de l'écorce (suber de bouleau)

15. Emulsion selon l'un des revendications 13 ou 14,
**caractérisée en ce que**
l'extrait de plantes contient au moins une des substances suivantes : bétuline, acide bétulinique, lupeol, erythrodiol, allobetuline, acide phellonique, hydroxilaktone, betulinaldehyde, β-amyrine, acide oléique, acide ursolique, betuline estérifiée et/ou β-sitosterol.

16. Emulsion selon l'un des revendications 13 à 15,
**caractérisée en ce que**
la fraction de triterpène et/ou de son dérivé atteint 2 à 10 % du poids total de l'émulsion.

17. Emulsion selon la revendication 16,
**caractérisée en ce que**
la fraction de triterpène et/ou de son dérivé atteint 2 à 3,5 % ou 3,5 à 10 % du poids total de l'émulsion.

18. Emulsion selon l'une des revendications 13 à 17,
**caractérisée en ce que**
l'huile est de l'huile d'avocat ou d'amande.

19. Emulsion selon l'une des revendications 13 à 18,
**caractérisée en ce que**
l'émulsion contient de plus au moins un agent de maintien d'humidité et/ou un épaississant.

20. Emulsion selon la revendication 19,
**caractérisée en ce que**
le moyen de maintien d'humidité est de la glycérine et/ou de l'urée, ajouté à une concentration de 3 à 10 %, de préférence 5 %, chacun, par rapport à la quantité d'émulsion.

21. °) Emulsion selon la revendication 19,
**caractérisée en ce que**
l'épaississant est un polysaccharide, ajouté à une concentration de 0,2 % à 2 %, de préférence 0,5 % par rapport à la quantité d'eau.

22. Emulsion selon la revendication 21,
**caractérisée en ce que**
le polysaccharide est de l'agar agar ou du carragenane.

23. Emulsion selon l'une des revendications 13 à 22,
**caractérisée en ce que**
l'émulsion contient 2 à 10 % d'extrait de la partie blanche d'écorce de bouleau, 20 à 30 % d'huile d'avocat, 10 à 20 % d'huile d'amande et 40 à 68 % d'eau.

24. Emulsion selon la revendication 23,
**caractérisée en ce que**
l'émulsion contient 4 % de l'extrait de la partie blanche de bouleau, 29,3 % d'huile d'avocat, 14,7 % d'huile d'amande et 52 % d'eau.

25. Emulsion selon l'une des revendications 13 à 22,
**caractérisée en ce que**
l'émulsion contient 2 à 10 % d'extrait de la partie blanche de l'écorce de bouleau, 20 à 30 % d'huile d'avocat, 10 à 20 % d'huile d'amande, 5 à 10 % d'agent de maintien d'humidité et 30 à 63 % d'eau.

26. Emulsion selon la revendication 25,
**caractérisée en ce que**
l'émulsion contient 4 % d'extrait de la partie blanche d'écorce de bouleau, 29,3 % d'huile d'avocat, 14,7 % d'huile d'amande, 5 % de glycérine et/ou 5 % d'urée et 42 à 47 % d'eau.

27. Emulsion selon l'une des revendications 13 à 26,
**caractérisée en ce que**
l'extrait de la partie blanche de l'écorce de bouleau contient au moins 80 % de bétuline, 10 % d'acide bétulinique au plus, 3 % de lupeol au plus et 4 % d'acide oléique au plus.

28. Procédé pour fabriquer une émulsion selon l'une des revendications 13 à 27,
présentant les étapes suivantes :
a) dispersion du triterpène dans une huile et/ou une graisse
b) émulsification par de l'eau
c) homogénéisation de l'émulsion jusqu'à la consistance nécessaire.

29. Procédé selon la revendication 28,
**caractérisé en ce que**
la dispersion et l'homogénéisation ont lieu dans un mélangeur d'homogénéisation.

30. Procédé selon l'une des revendications 28 ou 29,
**caractérisé en ce que**
à l'étape (b), est ajouté à l'eau un agent de maintien d'humidité, en particulier de la glycérine et/ou de l'urée, ou un épaississant, en particulier un polysaccharide.

31. Utilisation de l'émulsion selon l'une des revendications 13 à 27, pour fabriquer un cosmétique, de préférence sous la forme d'une pâte, d'une lotion, d'une crème, d'un gel ou d'un shampooing, en particulier pour application sur la peau, le cuir chevelu et/ou pour inhalation.

32. Utilisation de l'émulsion selon l'une des revendications 13 à 27, pour fabriquer un produit médicinal, de préférence sous la forme d'une pâte, d'une lotion, d'une crème, d'un gel, d'une gelée ou d'un shampooing, en particulier pour application sur la peau, le cuir chevelu et/ou pour inhalation, préférentiellement dans les altérations de la peau ou du cuir chevelu, avant tout dans les neurodermites, le psoriasis, l'eczéma séborrhéique, la mélanodermie, les précancers de la peau, ainsi que pour inhalation dan les affections asthmatiques et/ou substitution de glucocorticoïdes.
